# EUROPÄISCHE PATENTANMELDUNG

(11) **EP 2 072 023 A1**
(43) Veröffentlichungstag der Anmeldung: **24.06.2009**
(21) Anmeldenummer: 08022124.5
(22) Anmeldetag: 18.12.2008
(51) Int. Cl.: A61F 2/00

(54) **Flächiges Implantat, insbesondere zur Hernienversorgung**

(30) Priorität: 20.12.2007 DE 102007063214
(71) Anmelder: Aesculap AG, 78532 Tuttlingen (DE)
(72) Erfinder: Abele, Wolfgang, 78532 Tuttlingen/Donau (DE); Odermatt, Erich, 8200 Schaffhausen (CH)
(74) Vertreter: Patentanwälte Ruff, Wilhelm, Beier, Dauster & Partner

(57) **Zusammenfassung**

Die vorliegende Erfindung betrifft ein flächiges Implantat (10; 20; 30) in Form eines ersten textilen Flächengebildes (22; 32), das vollflächig und abdichtend beschichtet ist und eines zweiten mit dem ersten Flächengebilde (22; 32) in einer gemeinsamen Verbundebene verbundenen textilen Flächengebildes (24; 34), das frei von einer Beschichtung (26; 36) ist.

## Beschreibung

Die vorliegende Erfindung betrifft ein flächiges Implantat, insbesondere ein Herniennetz.

Die Versorgung einer Hernie bzw. eines Eingeweidebruchs gehört zu den wichtigsten Aufgabenbereichen in der visceralen oder parietalen Chirurgie. Hierbei handelt es sich im Allgemeinen um einen Austritt von Eingeweiden aus der Bauchhöhle durch eine angeborene oder erworbene Öffnung, die sogenannte Bruchpforte. Unter den äußeren Brüchen, bei denen stets der Bruchsack vom Bauchfell umschlossen wird, sind Leisten, Nabel und Narbenbrüche die häufigsten Formen. Ursachen für das Auftreten von Hernien sind vor allem Muskel- oder Bindegewebsschwächen. Diese können infolge von Überlastungen, altersbedingter Erschlaffung, ungenügender Narbenbildung nach einem operativen Eingriff oder einer angeborenen Schwächung der Bauchdecke entstehen.

Eine effektive Behandlung ist in den meisten Fällen durch einen operativen Eingriff möglich, bei dem der Bruchinhalt aus dem Bruchsack in den Bauch zurückverlagert und die Bruchpforte verschlossen wird. Bislang erfolgte der Verschluss der Bruchpforte konventionell mit Hilfe von Nahtmaterialien. Allerdings führte diese Art der chirurgischen Versorgung vor allem bei größeren Hernien zu einer nicht unerheblichen Bruchrezidivrate.

Deswegen werden in der modernen Hernienchirurgie zunehmend künstliche Verstärkungsmaterialien zur Bauchwandrekonstruktion verwendet. Hierbei sind vor allem Polypropylen- aber auch Polyesternetze von Bedeutung.

Obwohl die Verwendung solcher Netze offensichtlich zu einer deutlichen Verringerung der Rezidivrate geführt hat, sind diese Implantate vor allem in Hinblick auf post-chirurgische Verwachsungen mit dem Bauchraum nicht unproblematisch. Derartige Verwachsungen sind für die Betroffenen häufig sehr schmerzhaft und führen teilweise zu einer erheblichen Einschränkung der Beweglichkeit. Vor allem erhöhen sie aber das Risiko einer erneuten chirurgischen Intervention.

In der Zwischenzeit sind daher Hernienimplantate kommerziell erhältlich, die aufgrund ihrer Struktur Gewebeverwachsungen im Bauchraum vorbeugen. Dies kann beispielsweise durch eine einseitig mikroporöse Strukturierung des Implantats erreicht werden. Durch eine derartige Struktur kann eine zelluläre Kolonisierung auf der mikroporösen Seite des Implantats im Wesentlichen vermieden werden. Ein derartiges Implantat wurde von der Anmelderin selbst entwickelt. Diesbezüglich wird auf die DE 199 12 648 A1 verwiesen.

Weiterhin kommen Implantate zum Einsatz, weiche eine antiadhäsive Beschichtung auf der dem Bauchraum zugewandten Implantatseite auf-Beschichtung auf der dem Bauchraum zugewandten Implantatseite aufweisen. Die Beschichtungen können dabei aus einem elastomeren Material bestehen. Beispielsweise ist ein derartiges Implantat aus der WO 93/17635 A1 bekannt. Weitere beschichtete Hernienimplantate mit diversen Beschichtungsmaterialien sind aus den EP 0 797 962 B1, EP 1 317 227 B1 und EP 0 998 314 B1 bekannt.

Bei dem aus der EP 0 998 314 B1 bekannten Implantat handelt es sich um eine Verbundprothese, welche insbesondere in Form eines Abstandstextils vorliegen kann. Abstandstextilien sind allgemein aufwendig herzustellende Textilerzeugnisse, welche aufgrund ihrer steifen Eigenschaften zudem nur begrenzt zur Versorgung von Hernien geeignet sind.

Somit stellt sich die vorliegende Erfindung die Aufgabe, ein Implantat bereitzustellen, welches sich insbesondere zur Versorgung von Hernien eignet, ohne dass es zu post-chirurgischen Gewebeverwachsungen des Implantats mit dem Bauchraum kommt. Das Implantat soll weiterhin über ausreichend flexible Eigenschaften verfügen und möglichst einfach in seiner Herstellung sein.

Diese Aufgabe wird erfindungsgemäß gelöst durch ein flächiges Implantat in Form eines ersten textilen Flächengebildes, das vollflächig und abdichtend beschichtet ist und eines zweiten mit dem ersten Flächengebilde in einer gemeinsamen Verbundebene verbundenen textilen Flächengebildes, das frei von einer Beschichtung ist.

Durch die Erfindung wird ein Implantat bereitgestellt, welches sich insbesondere zur Versorgung von Hernien eignet, ohne dass es nach der Implantation zu post-chirurgischen Gewebeverwachsungen zwischen Weichgewebe aus dem Bauchraum und der dem Bauchraum zugewandten seite des Implantats kommt Die vollflächige und abdichtende Beschichtung des ersten textilen Flächengebildes verhindert eine zelluläre Kolonisierung oder Besiedelung dieser Implantatseite. Dadurch bleibt das Implantat auf seiner beschichteten Seite mit besonderem Vorteil beweglich. Die doppelt-textile Struktur des Implantats begünstigt weiterhin eine stärkere Infiltration des Implantats mit Körperzellen ausgehend von der Bauchdeckenseite. Dabei können die Körperzellen tiefer und insbesondere in größerer Anzahl in das Implantat eindringen, ohne dass hiervon die Beschichtung des ersten textilen Flächengebildes nachteilig beeinflusst wird. Dies unterstützt eine starke und sichere Verankerung des Implantats mit der Bauchdeckenwand.

Grundsätzlich können beide Flächenseiten des ersten textilen Flächengebildes vollflächig und abdichtend beschichtet sein. Vorzugsweise ist nur eine Flächenseite, insbesondere die zur Außenseite des Implantats gerichtete Flächenseite, des ersten textilen Flächengebildes vollflächig und abdichtend beschichtet.

In einer bevorzugten Ausführungsform sind die beiden textilen Flächengebilde nur stellenweise miteinander verbunden. Vorzugsweise weisen die beiden Flächengebilde Flächenabschnitte auf, an denen sie gegeneinander in der Verbindungsebene verschieblich sind. In dieser Ausführungsform kann das Implantat in geeigneter Weise auf im Körper auftretende Scherkräfte reagieren. Dadurch lässt sich die Elastizität des Implantats in besonders vorteilhafter Weise erhöhen.

In einer möglichen Ausführungsform sind die beiden textilen Flächengebilde an den Rändern miteinander verbunden. Vorzugsweise weisen die beiden Flächengebilde alternativ oder zusätzlich randferne Verbindungen auf. Dies hat den besonderen Vorteil, dass das Implantat vor seiner Implantation durch den Chirurgen auf eine geeignete Größe zurechtgeschnitten werden kann.

In einer weitergehenden Ausführungsform sind die beiden textilen Flächengebilde über linienförmige Verbindungen miteinander verbunden. Die linienförmigen Verbindungen können beispielsweise parallel zueinander verlaufend sein. Andere Verläufe sind erfindungsgemäß aber ebenso möglich. So können die linienförmigen Verbindungen beispielsweise einen serpentinenförmigen Verlauf aufweisen. Bevorzugt sind die beiden textilen Flächengebilde über punktförmige Verbindungen miteinander verbunden. Der Mindestabstand zwischen zwei Verbindungsstellen beträgt vorzugsweise ca. 2,5 cm. Allgemein kann das Implantat zwei- und/oder dreidimensionale Verbindungen aufweisen.

Grundsätzlich kommen zur Verbindung der beiden textilen Flächengebilde alle dem Fachmann geläufigen Verbindungstechniken in Frage, die auch miteinander kombiniert sein können. Bevorzugt sind die beiden Flächengebilde miteinander vernäht. Erfindungsgemäß kann es weiterhin vorgesehen sein, dass die beiden Flächengebilde miteinander verklebt sind. In einer weiteren Ausführungsform sind die beiden Flächengebilde miteinander verschweißt. Die beiden Flächengebilde können beispielsweise durch Ultraschall-Schweißen miteinander verschweißt sein. Die in diesem Abschnitt genannten Ausführungsformen ermöglichen mit besonderem Vorteil eine dauerhafte Verbindung der beiden textilen Flächengebilde und tragen damit zur weiteren Stabilität des erfindungsgemäßen Implantats bei.

In einer weitergehenden Ausführungsform weisen die beiden textilen Flächengebilde eine unterschiedliche Flächenausdehnung auf. Beispielsweise kann das erste Flächengebilde das zweite Flächengebilde überragen. Erfindungsgemäß ist es aber ebenso möglich, dass das zweite Flächengebilde aufgrund einer größeren Flächenausdehnung das erste Flächengebilde überragt.

Bevorzugt weist das erste Flächengebilde die Beschichtung auf einer Seite des Implantats auf, die vom zweiten Flächengebilde abweist. Die beschichtete Seite des ersten Flächengebildes ist nach der Implantation bevorzugt dem Bauchraum zugewandt. Die Beschichtung wirkt in vorteilhafter Weise als eine Art Barriere, die eine post-chirurgische Verwachsung zwischen Weichgewebe des Bauchraums und dem Implantat verhindert. Das erste textile Flächengebilde besitzt durch die Beschichtung vorzugsweise eine glatte Oberfläche. Dadurch kann eine zelluläre Kolonisierung der beschichteten Implantatseite und damit das Auftreten von Weichgewebeadhäsionen verhindert werden.

Grundsätzlich können die beiden textilen Flächengebilde in Form von Maschenwaren, Geweben, Vliesen oder Gelegen vorliegen. Als Maschenwaren kommen insbesondere Gewirke in Betracht. Erfindungsgemäß ist es insbesondere vorgesehen, dass zumindest ein Flächengebilde des Implantats ein Gewirk ist. Vorzugsweise sind beide textilen Flächengebilde Gewirke.

In einer weiteren Ausführungsform weist das zweite textile Flächengebilde für Körperzellen hintergreifbare Stellen auf. Insbesondere kann das zweite textile Flächengebilde texturierte Garne, Flottungen, Veloursschlingen und/oder Flore aufweisen. Erfindungsgemäß kann es insbesondere vorgesehen sein, dass aus Veloursschlingen hervorgehende Flore unterschiedliche Längen aufweisen.

Das zweite textile Flächengebilde kann in einer weitergehenden Ausführungsform als Velours vorliegen. Bei dem Velours kann es sich um ein Einfach- oder Doppelvelours handeln. Im Falle eines Doppelvelours kann dieser auf beiden Seiten des zweiten Flächengebildes unterschiedliche Polhöhen aufweisen.

In einer bevorzugten Ausführungsform sind die textilen Flächengebilde des Implantats aus einem nicht resorbierbaren Material, insbesondere Fadenmaterial, gebildet. Bevorzugt handelt es sich bei dem nicht resorbierbaren Material um ein Polyolefin und/oder Polyester. Als Polyester kommt beispielsweise Polyethylenterephthalat in Frage. Bei den Polyolefinen kann es sich beispielsweise um Polypropylen, Polyvinylidendifluorid (PVDF) und/oder Polytetrafluorethylen handeln, wobei Polypropylen als Material bevorzugt ist.

In einer weiteren Ausführungsform sind die textilen Flächengebilde aus einem resorbierbaren Material, insbesondere Fadenmaterial, gebildet. Als resorbierbare Materialien kommen vor allem Polymere auf der Basis von Hydroxycarbonsäureeinheiten in Betracht. Geeignete resorbierbare Materialien sind daher insbesondere Polymere aus Lactid, Glykolid, ε-Caprolacton, Trimethylencarbonat, p-Dioxanon, Hydroxybuttersäure und/oder Copolymere davon.

Sofern eine Teilresorbierbarkeit des erfindungsgemäßen Implantats erwünscht ist, können die textilen Flächengebilde auch aus einem resorbierbaren Material und einem nicht resorbierbaren Material gebildet sein. Beispielsweise können beide Flächengebilde jeweils aus einem resorbierbaren und einem nicht resorbierbaren Anteil bestehen. Alternativ können das erste Flächengebilde aus einem resorbierbaren Material und das zweite Flächengebilde aus einem nicht resorbierbaren Material oder umgekehrt bestehen. Beispielsweise kann das erste Flächengebilde aus Polyglykolid oder einem Copolymer davon und das zweite Flächengebilde aus Polypropylen und/oder Polyethylenterephthalat bestehen. Die umgekehrte Materialkombination kann erfindungsgemäß ebenso vorgesehen sein.

Grundsätzlich können die textilen Flächengebilde aus monofilen und/oder multifilen Fäden, insbesondere Garnen, bestehen. Die Fäden können insbesondere glatt oder texturiert sein. Vorzugsweise besteht mindestens ein Flächengebilde, insbesondere beide Flächengebilde, aus Monofilamenten bzw. monofilen Fäden. Die textilen Flächengebilde können zumindest teilweise, vorzugsweise vollständig, aus geflochtenen Fäden, beispielsweise aus Polyglykolid, bestehen. Die Fäden können weiterhin mit einem resorbierbaren Material beschichtet sein. Bezüglich möglicher Beschichtungsmaterialien wird auf die weitere Beschreibung Bezug genommen.

In einer weitergehenden Ausführungsform weist zumindest ein textiles Flächengebilde des Implantats Fäden mit einer Bikomponentenstruktur, insbesondere mit einer Kern-Mantel-Struktur, auf. Beispielsweise können die Fäden aus einem nicht resorbierbaren Kernpolymer, beispielsweise Polypropylen, und einem resorbierbaren Mantelpolymer bestehen. Die Verwendung von Bikomponentenfäden mit resorbierbaren Mantelpolymeren hat den Vorteil, dass das Implantat nach Resorption des Mantelpolymers eine insgesamt größere Beweglichkeit zeigt. Zudem wird der Materialeintrag in den Körper eines Patienten durch die Resorbierbarkeit des Mantelpolymers verringert, wodurch sich weniger, Narbengewebe ausbilden kann. Als geeignete Mantelpolymere kommen insbesondere Polyglykolid, Polylactid, Polyhydroxybuttersäure, Poly-ε-Caprolacton, Polytrimethylencarbonat, Polydioxanon und/oder Copolymere davon in Frage. Bezüglich weiterer Einzelheiten und Merkmale zu möglichen Kern- bzw. Mantelpolymeren wird auf die bisherige Beschreibung verwiesen.

In einer besonders bevorzugten Ausführungsform besitzen die textilen Flächengebilde eine Netzstruktur. Die offene Struktur des textilen Netzes begünstigt auf der nicht beschichteten Seite des Implantats das Einwachsen von Körperzellen, so dass auf diese Weise eine Verankerungsmöglichkeit des Implantats mit der Abdominalwand (Bauchwand) möglich ist. Die textilen Flächengebilde besitzen in einer weitergehenden Ausführungsform eine lichte Maschenweite von 0,3 mm bis 6 mm, insbesondere 1 mm bis 4 mm.

Weiterhin ist es bevorzugt, dass die textilen Flächengebilde flexibel sind. Hierdurch können Bewegungen in der Bauchregion, welche vor allem durch die Bauchmuskulatur verursacht werden, in vorteilhafter Weise kompensiert werden. Erfindungsgemäß ist es insbesondere vorgesehen, dass die textilen Flächengebilde einschließlich der Beschichtung dehnbar, vorzugsweise isometrisch dehnbar, sind.

Bevorzugt weisen die textilen Flächengebilde in Längs- und Querrichtung eine unterschiedliche Reißdehnung von maximal 15% auf. In einer weiteren Ausführungsform besitzen die textilen Flächengebilde eine lineare Reißkraft, die größer als 10 N/cm ist, insbesondere ca. 16 N/cm beträgt.

In einer weiteren Ausführungsform weist das erste textile Flächengebilde eine kleinere Porenweite als das zweite textile Flächengebilde auf. Dadurch ist eine abdichtende Beschichtung des ersten Flächengebildes besonders einfach zu realisieren. Die größere Porenweite des zweiten Flächengebildes begünstigt seine zelluläre Kolonisierung oder Besiedelung und damit die Fixierung des Implantats an der Bauchwand. Bevorzugt besitzt mindestens ein textiles Flächengebilde, vorzugsweise beide Flächengebilde, eine Porenweite von weniger als 1,7 mm. Insbesondere weist das erste textile Flächengebilde eine Porenweite < 2 mm, vorzugsweise zwischen 0,2 und 1,5 mm, auf. Das zweite textile Flächengebilde weist bevorzugt eine Porenweite von 2 mm bis 5 mm, insbesondere 3 mm bis 4 mm, auf.

In einer möglichen Ausführungsform weisen die beiden textilen Flächengebilde die gleiche Porenweite auf.

In einer weiteren Ausführungsform besitzt mindestens ein Flächengebilde, vorzugsweise jeweils beide Flächengebilde, ohne Berücksichtigung einer Beschichtung ein Flächengewicht < 60 g/m², insbesondere < 40 g/m². Bevorzugt besitzt die Beschichtung ein Oberflächengewicht < 100 g/m², insbesondere < 60 g/m².

Die Beschichtung weist in einer weiteren Ausführungsform eine Dicke von ca. 1 mm, insbesondere < 100 µm, auf. Bevorzugt besitzt die Beschichtung eine Dicke von 30 % bis 70 %, insbesondere 40 % bis 70 %, der Gesamtdicke des Implantats. Insbesondere weist die Beschichtung einen Anteil im Implantat von 30 Gew.-% bis 80 Gew.-%, insbesondere 30 Gew.-% bis 60 Gew.-%, vorzugsweise 30 Gew.-% bis 40 Gew.-%, auf, bezogen auf das Gesamtgewicht des Implantats. Erfindungsgemäß kann es weiterhin vorgesehen sein, dass die Beschichtung an das Implantat über eine Tiefe von < 400 µm, insbesondere 200 µm bis 300 µm, gemessen von der äußeren Oberfläche der Beschichtung, gebunden ist.

Bevorzugt weist die Beschichtung des ersten textilen Flächengebildes ein resorbierbares Material auf. Vorzugsweise besteht die Beschichtung aus einem resorbierbaren Material. Bei dem resorbierbaren Material kann es sich um resorbierbare Polymere, insbesondere auf der Basis von Hydroxycarbonsäuren, handeln. Geeignete Beispiele sind die bereits erwähnten Polymere Polyglykolid, Polylactid, Poly-ε-Caprolacton, Polytrimethylencarbonat, Poly-p-dioxanon, Polyhydroxybuttersäure und/oder Copolymere davon. Ein weiteres geeignetes Material ist Polyvinylalkohol. Bevorzugt weist die Beschichtung ein biologisches Material, insbesondere Proteine, auf. Das biologische Material kann xenogenen Ursprungs sein. Beispielsweise kann das biologische Material bovinen, porcinen und/oder equinen Ursprungs sein. Weiterhin kann das biologische Material rekombinant sein. Erfindungsgemäß ist es aber auch möglich, dass es sich bei dem biologischen Material um humane Proteine, insbesondere autologe Proteine, handelt. Bei dem biologischen Material kann es sich insbesondere um extrazelluläre Matrixproteine oder um Serumproteine handeln. Als geeignete biologische Materialien kommen vor allem Collagene, Gelatinen und/oder Albumine in Betracht. Vorzugsweise besteht die Beschichtung des Implantats aus Gelatine. Die Gelatine weist in vorteilhafter Weise eine Resorptionszeit von mindestens 7 Tagen auf. Nach dieser Zeit hat sich gewöhnlich neues Peritoneum (Pseudoperitoneum) über dem Implantat gebildet, welches nun die Funktion der Beschichtung übernimmt und als natürliche Schutzbarriere gegen Gewebeverklebungen mit dem Bauchraum wirkt. Die Gelatine kann ein Molekulargewicht von 50 kilo Dalton (kDa) bis 200 kilo Dalton (kDa), insbesondere von 100 kilo Dalton (kDa), besitzen. Bei der Gelatine kann es sich weiterhin um chemisch denaturiertes Collagen oder thermisch denaturiertes Collagen handeln. Bevorzugt leitet sich die Gelatine von thermisch denaturiertem Collagen ab. Die Gelatine kann zudem vernetzt, insbesondere chemisch vernetzt, sein. Beispielsweise kann die Gelatine mit einem Diisoyanat vernetzt sein. Diesbezüglich wird auf die EP 0 237 037 A2 Bezug genommen, deren Offenbarungsgehalt durch ausdrückliche Bezugnahme zum Inhalt dieser Beschreibung gemacht wird.

Die Beschichtung kann weiterhin ein Hydrogel sein. Bevorzugt ist die Beschichtung in Form einer Folie oder eines Films ausgebildet. Bei einer weiteren Ausführungsform ist das erste textile Flächengebilde oberflächenbehandelt, insbesondere durch eine Oberflächenbehandlung aktiviert, um eine bessere Haftung der Beschichtung auf dem ersten textilen Flächengebilde zu erzielen. Beispielsweise kann das erste textile Flächengebilde mittels Plasma behandelt sein.

In einer weiteren Ausführungsform enthält das Implantat antimikrobiotische Wirkstoffe, insbesondere Antibiotika und/oder Desinfektiva. Die Ausrüstung des Implantats mit Antibiotika dient insbesondere der Vorbeugung von Infektionen. Zur Prophylaxe und Therapie mit Antibiotika finden im Bereich der Chirurgie beispielsweise Cefalosporine, Penicilline oder Aminoglykoside Anwendung. Bei den Cefalosporinen kann es sich beispielsweise um Cefazolin, Cefamandol und/oder Netilmizin handeln. Als geeignete Penicilline kommen vor allem Oxacillin, Mezlocillin, Tetrazyclin und/oder Metronidazol in Betracht. Geeignete Aminoglykoside stellen Gentamyzin, Neomyzin und/oder Rifampicin dar. Des Weiteren kann das Implantat auch andere Wirkstoffe, insbesondere Wachstumsfaktoren, enthalten.

Weiterhin kann es erfindungsgemäß vorgesehen sein, dass das Implantat in sterilisierter Form vorliegt. Als geeignete Sterilisierverfahren kommen die üblichen physikalischen oder chemischen Methoden zum Inaktivieren von Mikroorganismen in Betracht. Ein mögliches Sterilisierungsverfahren betrifft die Behandlung mit ionisierender Strahlung, beispielsweise mit β-Strahlen oder γ-Strahlen, insbesondere in einem Bereich von 5 kGy bis 38 kGy. Strahlendosen in einem Bereich von 18 kGy bis 30 kGy sind beispielsweise für eine Sterilisierung von Polyester-Grundmaterialien geeignet. Ein bevorzugtes Sterilisationsverfahren ist die Gassterilisierung, beispielsweise Ethylenoxid- und/oder Formaldehyd-Begasung. Eine weitere mögliche Sterilisationstechnik ist die Plasmasterilisierung. Die Plasmasterilisierung kann mit oder ohne Wasserstoffperoxid betrieben werden. Eine Sterilisierung des erfindungsgemäßen Implantats mittels Dampfbehandlung ist grundsätzlich möglich.

Des Weiteren betrifft die vorliegende Erfindung auch die Verwendung des Implantats in der Chirurgie, insbesondere zur Behandlung von Wanddefekten in Körperhöhlen, vorzugsweise von Bauchwand-, Thorax- und/oder Unterleibsdefekten. Zu diesem Zweck kann das erfindungsgemäße Implantat auf eine gewünschte Größe und Form zurechtgeschnitten werden.

Mit Vorteil kann das Implantat in zweckmäßiger Dimension zugeschnitten, gebrauchsfertig und insbesondere in geeigneter Weise verpackt vorliegen.

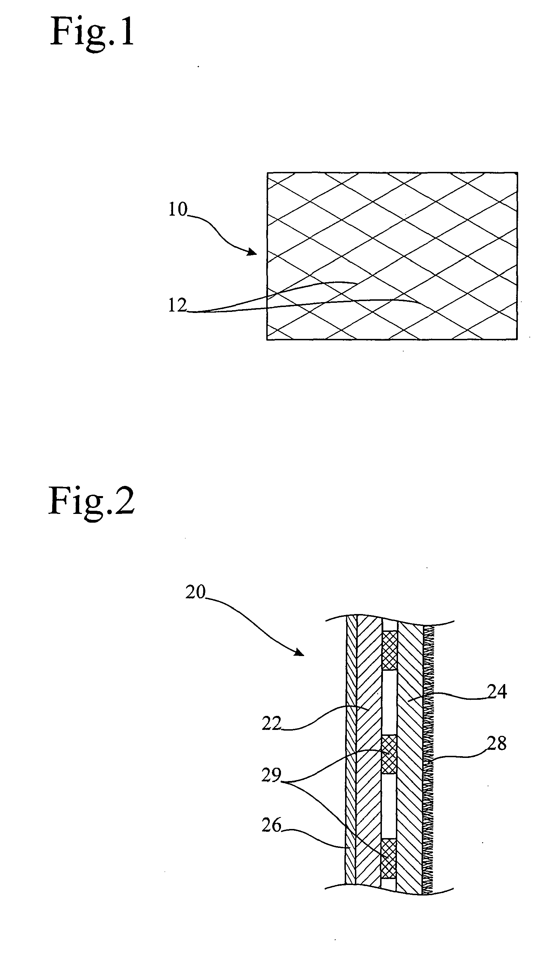

Weitere Einzelheiten und Merkmale der Erfindung ergeben sich aus der nachfolgenden Beschreibung bevorzugter Ausführungsformen anhand der Figuren 1, 2 und 3 in Kombination mit den Unteransprüchen. In diesen Ausführungsformen können einzelne Merkmale der Erfindung allein oder in Kombination mit anderen Merkmalen verwirklicht sein. Die beschriebenen bevorzugten Ausführungsformen sind lediglich als beschreibende, keineswegs als in irgendeiner Weise limitierende Offenbarung zu verstehen. Die Figuren 1, 2 und 3 werden hiermit durch ausdrückliche Bezugnahme zum Inhalt dieser Beschreibung gemacht.

In den Figuren ist schematisch gezeigt:
- Figur 1:: die Draufsicht auf ein erfindungsgemäßes Implantat,
- Figur 2:: die Seitenansicht einer Ausführungsform des erfindungsgemäßen Implantats,
- Figur 3:: die Seitenansicht einer weiteren Ausführungsform des erfindungsgemäßen Implantats.

### Figurenbeschreibung

In Figur 1 wird schematisch die Draufsicht auf ein flächiges Implantat 10 gemäß der vorliegenden Erfindung gezeigt. Das Implantat 10 ist aus zwei textilen Netzen aufgebaut, die durch linienförmige Verklebungen 12 miteinander verbunden sind.
In Figur 2 ist schematisch die Seitenansicht eines erfindungsgemäßen Implantats 20 gezeigt. Das Implantat 20 ist aus den in Form von textilen Netzen vorliegenden Flächengebilden 22 und 24 aufgebaut, die über punktuelle Verklebungsstellen 29 miteinander verbunden sind. Das Netz 22 weist auf einer Flächenseite eine vollflächige und abdichtende Beschichtung 26 aus Gelatine auf. Die Gelatine-Beschichtung 26 wirkt als Barriere für Körperzellen, insbesondere Gewebezellen aus dem peritonealen Körperbereich. Dadurch kann mit besonderem Vorteil ein Eindringen von Körperzellen auf der beschichteten Seite des Implantats 20 während der Entstehung eines Pseudoperitoneums im Wesentlichen verhindert werden. Bei dem Netz 24 handelt es sich vorzugsweise um ein Einfachvelours-Netz, dessen Veloursfäden 28 zur Außenseite des Implantats 20 gerichtet sind. Die Veloursfäden 28 stellen grundsätzlich für Körperzellen hintergreifbare Strukturen dar und fördern damit die zelluläre Besiedelung und Infiltration des Implantats 20 ausgehend von seiner unbeschichteten Seite. Das Implantat 20 eignet sich in besonderer Weise als Hernienimplantat, dessen Implantation zweckmäßigerweise derart durchgeführt wird, dass die Flächenseite mit der Beschichtung 26 dem Bauchraum und die Flächenseite mit den Veloursfäden 28 der Bauchwand zugewandt sind.

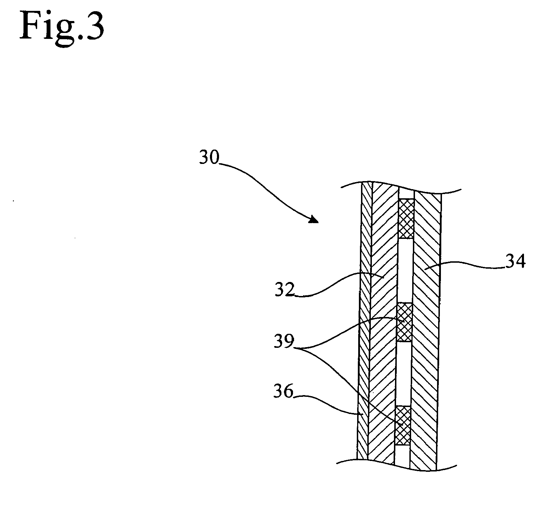

In Figur 3 ist schematisch die Seitenansicht eines weiteren erfindungsgemäßen Implantats 30 gezeigt. Das Implantat 30 ist aus den als textile Netze vorliegenden Flächengebilden 32 und 34 aufgebaut. Die textilen Netze 32; 34 sind über punktuelle Verklebungsstellen 39 miteinander verbunden. Das Netz 32 ist aus Bikomponentenfasern hergestellt. Diese weisen eine Kern-Mantel-Struktur auf, wobei der Faserkern aus Polypropylen und der Mantel (Ummantelung der Fasern) aus Polyglykolid bestehen. Die zur Außenseite des Implantats 30 gerichtete Flächenseite des Netzes 32 ist mit einer Folie 36 aus Polyglykolid beschichtet. Die Polyglykolid-Folie 36 wirkt als Barriere für Körperzellen, insbesondere Gewebezellen aus dem peritonealen Körperbereich. Dadurch kann sich ein Pseudoperitoneum an der beschichteten Seite des Implantats 30 ausbilden, ohne dass es zu Gewebeverwachsungen mit dem Bauchraum kommt. Bei dem Netz 34 handelt es sich um ein großporiges Netz, dessen Porosität allgemein die zelluläre Besiedelung und Infiltration des Implantats 30 ausgehend von seiner unbeschichteten Seite fördert. Das Implantat 30 eignet sich ebenso besonders als Hernienimplantat, dessen Implantation zweckmäßigerweise derart durchgeführt wird, dass die Flächenseite mit der Folie 36 dem Bauchraum und die gegenüberliegende Flächenseite des Implantats der Bauchwand zugewandt sind.

## Patentansprüche

1. Flächiges Implantat (10; 20; 30) in Form eines ersten textilen Flächengebildes (22; 32), das vollflächig und abdichtend beschichtet ist und eines zweiten mit dem ersten Flächengebilde (22; 32) in einer gemeinsamen Verbundebene verbundenen textilen Flächengebildes (24; 34), das frei von einer Beschichtung (26; 36) ist.

2. Flächiges Implantat (10; 20; 30) nach Anspruch 1, **dadurch gekennzeichnet, dass** die beiden Flächengebilde (22; 24; 32; 34) nur stellenweise miteinander verbunden sind.

3. Flächiges Implantat (10; 20; 30) nach Anspruch 1 oder 2, **dadurch gekennzeichnet, dass** die beiden Flächengebilde (22; 24; 32; 34) Flächenabschnitte aufweisen, an denen sie gegeneinander in der Verbindungsebene verschieblich sind.

4. Flächiges Implantat (10; 20; 30) nach einem der vorhergehenden Ansprüche, **dadurch gekennzeichnet, dass** die beiden Flächengebilde (22; 24; 32; 34) an den Rändern miteinander verbunden sind.

5. Flächiges Implantat (10; 20; 30) nach einem der vorhergehenden Ansprüche, **dadurch gekennzeichnet, dass** die beiden Flächengebilde (22; 24; 32; 34) über randferne Verbindungen miteinander verbunden sind.

6. Flächiges Implantat (10; 20; 30) nach einem der vorhergehenden Ansprüche, **dadurch gekennzeichnet, dass** die beiden Flächengebilde (22; 24; 32; 34) über linienförmige Verbindungen (12) miteinander verbunden sind.

7. Flächiges Implantat (10; 20; 30) nach einem der vorhergehenden Ansprüche, **dadurch gekennzeichnet, dass** die beiden Flächengebilde (22; 24; 32; 34) über punktförmige Verbindungen (29; 39) miteinander verbunden sind.

8. Flächiges Implantat (10; 20; 30) nach einem der vorhergehenden Ansprüche, **dadurch gekennzeichnet, dass** das erste Flächengebilde (22; 32) die Beschichtung (26; 36) auf einer Seite aufweist, die vom zweiten Flächengebilde (24; 34) abweist, wobei das erste Flächengebilde (22; 32) durch die Beschichtung (26; 36) eine glatte Oberfläche besitzt.

9. Flächiges Implantat (10; 20; 30) nach einem der vorhergehenden Ansprüche, **dadurch gekennzeichnet, dass** mindestens ein, vorzugsweise beide, Flächengebilde (22; 24; 32; 34) Gewirke sind.

10. Flächiges Implantat (10; 20; 30) nach einem der vorhergehenden Ansprüche, **dadurch gekennzeichnet, dass** das zweite textile Flächengebilde (24; 34) für Körperzellen hintergreifbare Stellen aufweist.

11. Flächiges Implantat (10; 20; 30) nach einem der vorhergehenden Ansprüche, **dadurch gekennzeichnet, dass** das zweite textile Flächengebilde (24; 34) texturierte Garne, Flottungen, Veloursschlingen und/oder Flore (28) aufweist.

12. Flächiges Implantat (10; 20; 30) nach einem der vorhergehenden Ansprüche, **dadurch gekennzeichnet, dass** das zweite textile Flächengebilde (24; 34) als Velours, insbesondere als Einfachvelours, vorliegt.

13. Flächiges Implantat (10; 20; 30) nach einem der vorhergehenden Ansprüche, **dadurch gekennzeichnet, dass** die textilen Flächengebilde (22; 24; 32; 34) eine Netzstruktur besitzen.

14. Flächiges Implantat (10; 20; 30) nach einem der vorhergehenden Ansprüche, **dadurch gekennzeichnet, dass** das erste textile Flächengebilde (22; 32) eine kleinere Porenweite als das zweite textile Flächengebilde (24; 34) aufweist, wobei das erste textile Flächengebilde (22; 32) eine Porenweite < 2 mm, vorzugsweise zwischen 0,2 und 1,5 mm, aufweist und das zweite textile Flächengebilde (24; 34) eine Porenweite von 2 mm bis 5 mm aufweist.

15. Flächiges Implantat (10; 20; 30) nach einem der vorhergehenden Ansprüche, **dadurch gekennzeichnet, dass** die Beschichtung (26; 36) aus einem resorbierbaren Material, insbesondere Polyvinylalkohol, Albumin und/oder Gelatine besteht.
